# EUROPEAN PATENT SPECIFICATION

(11) **EP 4 094 741 B1**
(45) Date of publication and mention of the grant of the patent: **28.05.2025**
(21) Application number: 21744891.9
(22) Date of filing: 25.01.2021
(51) Int. Cl.: A61H 1/02, A63B 23/04, A61H 9/00

(54) **EQUIPMENT FOR PASSIVE MOVEMENT OF THE KNEE JOINT**
VORRICHTUNG ZUR PASSIVEN BEWEGUNG DES KNIEGELENKS
ÉQUIPEMENT POUR LA MISE EN MOUVEMENT PASSIVE DE L'ARTICULATION DU GENOU

(30) Priority: 23.01.2020 BR 102020001512
(43) Date of publication of application: 30.11.2022
(73) Proprietor: BIOMOTION INDÚSTRIA E COMÉRCIO DE EQUIPAMENTOS PARA REABILITAÇÃO LTDA., 88015-310 - Florianópolis (BR)
(72) Inventor: PIERRI, Carlos Alberto Atherinos, 88090-100 Florianópolis (BR); BONA, Daniel Dezan De, 88063-200 Florianópolis (BR); CASTRO, Marcelo Peduzzi De, 88063-600 Florianópolis (BR)
(74) Representative: Girardi, Giovanna Paola
(86) International application number: PCT/BR2021/050038
(87) International publication number: WO 2021/146790

(56) References cited:
- EP-B1- 0 809 478
- BR-A2- PI1 015 791
- CN-A- 105 796 284
- CN-A- 110 604 673
- DE-C- 67 282
- US-A- 4 603 687
- US-A- 4 665 899
- US-A- 5 228 432
- US-A- 5 325 849
- US-A- 5 325 849
- US-A1- 2006 064 044
- US-A1- 2012 232 438
- US-A1- 2014 094 721
- US-B2- 10 420 691
- US-B2- 10 420 691
- US-B2- 9 125 789

## Description

The present patent document relates to automatic equipment for performing orthopedic and physiotherapeutic procedures for passive motion of the knee joint (patellofemoral and tibiofemoral).

The knee is formed by two joint surfaces, the patellofemoral joint and the medial and lateral tibiofemoral joint. The patellofemoral joint is formed by the frontal and distal joint surface of the femur, called trochlea and by the kneecap, which encases into and slides along the trochlea. In this joint there are lower-upper and medial-lateral displacement motions. The tibiofemoral joint is formed by the distal end of the femur and by the proximal end of the tibia. In this joint there are three pairs of motions, the flexion and extension (sagittal plane; medial-lateral axis of the knee), the internal and external rotation (transverse plane; axial or longitudinal axis) and the varus and valgus (frontal plane; anteroposterior axis). During knee motions, there is a combination of these joint displacements. For example, during extension of the knee (putting the leg straight), the external rotation of the knee and the upper sliding of the kneecap occur simultaneously.

The knee is a joint with high incidence of lesions, and among the treatment possibilities, multidirectional patellar mobilization is a frequently indicated therapeutic conduct. There are three procedures commonly used in clinical practice for recovery of the knee function: Immobilization, Motion or Passive Mobilization, and active Motion. Immobilization is necessary in some initial treatment phases, where, due to state of tissue healing, there can be no joint motion.

The motion or passive mobilization relates to the procedures applied to the patient while he/she is at rest, that is, the motion is performed by the physiotherapist or by means of an external device. The term passive motion is used when greater amplitudes are used, for example, flexion and extension of the knee. Whereas the term passive mobilization refers to motions of lesser joint amplitude, such as patellar sliding. Lastly, active motion relates to the motion performed actively by the patient, where the main objective is to produce motion by means of muscular contraction performed actively by the patient.

The passive tibiofemoral motion and patellar mobilization are therapeutic procedures classified in the clinical ambit as manual therapy technique, and are commonly recommended and applied during rehabilitation. Several studies have presented a series of clinical benefits after applying manual therapy, including patellar mobilization and passive tibiofemoral motion, in patients with dysfunctions in the knee (Anwer, Alghadir, Zafar, & Brismee, 2018; Deyle *et al.,* 2005; Deyle *et al.,* 2000). For example, two clinical assays (prospective monitoring study with two random intervention groups) showed that the use of manual therapy associated to exercise presents greater decrease of pain in the knee and functional improvement when compared to two different intervention approaches: placebo ultrasound (Deyle *et al.,* 2000) and home exercise program (Deyle *et al.,* 2005).

A systematic review accompanied by meta-analysis (Anwer *et al.,* 2018), comprising 494 patients with knee osteoarthrosis showed advantages of intervention with manual therapy compared to intervention exclusively with exercise. The patients who received only manual therapy presented in the short term greater reduction in pain, improvement in function and physical performance compared to the exercise group. Patellar mobilization and passive tibiofemoral motion were among the procedures most emphasized (Anwer *et al.,* 2018).

Patellar mobilization and passive tibiofemoral motion are among procedures most recommended for treating patients with pain in the knee associated to hypo-patellar and tibiofemoral mobility (Mullaney & Fukunaga, 2016). These motions are typically applied by the physiotherapist. However, some of the components of the patellar mobilization can be taught to the patient to be performed at home. Patellar self-mobilization appears to be an effective method since it allows a high dose of patellar mobilization, performed by the very patient (Mullaney & Fukunaga, 2016). The kneecap is moved as far as the restriction region and oscillatory motions or pressure maintained are applied (Mullaney & Fukunaga, 2016). Passive tibiofemoral motion is harder for the patient to be able to perform alone at home. One option is to teach family members. But both patellar mobilization and passive tibiofemoral motion are hard to perform at home, chiefly due to the presence of pain, insecurity and the technique which is hard to understand.

One of the main concerns after surgical intervention in the knee is the decrease in patellar and tibiofemoral mobility (Harrison *et* al., 2013; Wilk, Macrina, & Reinold, 2010). On average, a kneecap moves 1cm medially (confidence interval of 95% from 0.8cm to 1.4cm) and 1.2cm laterally (confidence interval of 95% from 0.7cm to 1.8cm) (Ota, Nakashima, Morisaka, Ida, & Kawamura, 2008). The reduction of patellar mobility is associated with excessive proliferation of healing tissue (arthrofibrosis) and adherence between different tissue layers (Wilk *et al.,* 2010). As a consequence, there is a decrease in the amplitude of tibiofemoral motion of the knee and decrease in the recruitment ability of the muscles of the quadriceps, essential conditions for adequate functioning of the knee joint.

The patellar mobilization and the passive tibiofemoral motion are considered the main therapeutic procedures in the sense of avoiding hypo-patellar mobility and decreasing the amplitude of motion of the knee (Reinold, Wilk, Macrina, Dugas, & Cain, 2006; Tyler & Lung, 2012; Wilk, Davies, Mangine, & Malone, 1998; Wilk *et al.,* 2010). Emphasis should be placed on the patellar mobilization to be performed in all the directions (lower, upper, medial and lateral), so that the passive tibiofemoral motion is performed up to the maximum amplitude available, and in high doses, covering the application several times per day (Reinold *et al.,* 2006; Tyler & Lung, 2012). Additionally, mobilization and passive joint motion appear to influence neural excitability associated to pain, promoting relief from pain (Courtney, Steffen, Fernandez-de-Las-Penas, Kim, & Chmell, 2016).

Today the patellar mobilization and passive tibiofemoral motion procedure is performed manually by physiotherapists who work with clinical rehabilitation or by the patient him/herself upon professional recommendation. The clinical indication for carrying out the mobilization and passive motion procedure may vary from a few days up to several weeks, with daily intervals of 4 to 5 times in routines from 5 to 15 minutes each (Reinold *et al.,* 2006; Tyler & Lung, 2012). The purpose of all this procedure is to improve/maintain the kneecap and/or to decrease the rigidity of the motion. Being a lengthy procedure, and applied during the course of the whole day, the physiotherapist is unable to accompany the patient for the entire rehabilitation process, placing the responsibility upon the individual and his/her family member/carers to carry out the procedures. However, the patients and their carers are not always proficient at performing said procedure, either through concern about handling the recently-operated knee or on which he/she feels pain, or from forgetfulness. Furthermore, self-mobilization appears not to allow the necessary relaxation of the quadriceps musculature, especially the rectus femoris muscle, causing protective contraction. As a consequence, this muscle causes compression of the kneecap and decreases its ability to move.

Since there is a strong dependency on the skill of the professional, or of the patient in carrying out the treatment adequately and with the necessary frequency, this treatment ends up not achieving the desired result, whereby contributing to the post-operation complications referred to.

Accordingly, in order to solve the problems cited above, eliminating the factor of uncertainty in the efficiency of the treatment owing to the dependency on the professional's skill, various semiautomatic and automatic apparatuses have been developed to perform the physiotherapeutic treatments in the knee area, without the need for constant professional supervision.

From prior art, it is possible to cite document EP2114322, which describes a constructive arrangement for a knee immobilizer, with protection accessory for the kneecap; and also the document JP3693368 describes a knee brace that has one or more inflatable cushions to adjustably provide safe and comfortable support for the strap pressed against the leg; and also the document EP2825139 describes a device to protect the knee and the kneecap the enables and accompanies the active motion of the knee, and includes a contact cushion for protecting the kneecap while the knee is moved in flexion and extension.

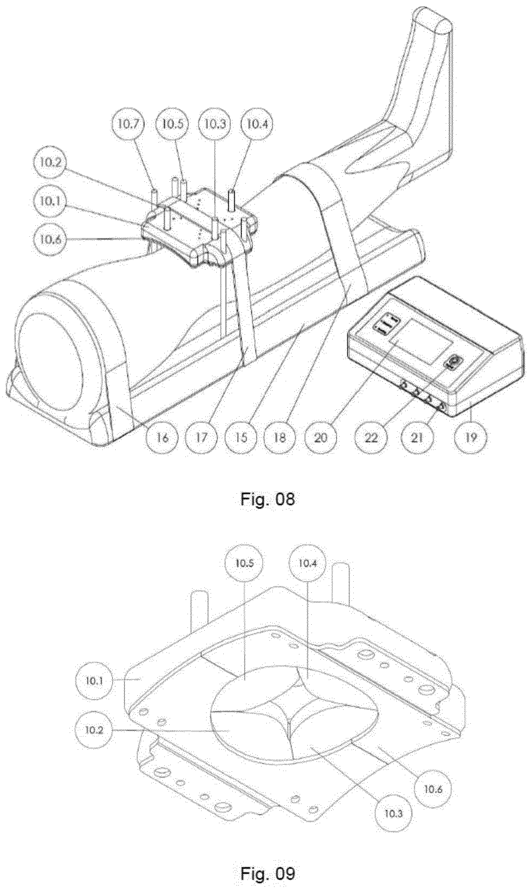

Another example is described in the CN document 110604673 which describes a lower limb joint healing device and a patella loosening instrument. The Chinese document indicates, through figures 8 and 9, that the mechanism for moving the patella is like a clamp that must be positioned around the patella to then make multidirectional movements but does not make it clear how it performs this function. Advantageously, the proposed invention comprises a pneumatic patella movement system, which allows, in addition to flexion and extension, rotation in the longitudinal axis of the distal segment (patient's leg).

Today, the most common devices to control and assure immobilization are braces. Besides immobilizing the joint, some braces are also used to enable controlled motion, preventing undesirable amplitudes. For example, there are braces that prevent the knee from being moved at an amplitude greater than 30 degrees, so the device blocks the knee when it reaches this range of motion.

In general, the technologies developed to date focused on eliminating the uncertainties and problems in physiotherapeutic treatment of the knee, caused by the imprecision in performing the treatment, either by the patient or by the professional. Such uncertainty cannot be controlled with efficiency when it depends on the human factor involved.

The challenge in the evolution of these technologies relates to the difficulty in replacing the human operator in the treatment that requires mobilization of the knee joint as a whole, as well as reliable mobilization of the kneecap because it refers to a delicate movement, being in a hard-to-access place and requiring a steady grip, slippage errors can easily occur, which adversely affects the treatment. If we also consider the combination of other motions, the difficulty of correct application of the procedure increases.

It is the object of the present invention to provide a device for performing multidirectional mobilization of the knee joint - both patellofemoral and tibiofemoral - in an automated and non-invasive manner, for post-surgery rehabilitation treatment and conservative (non-surgical) treatment of the knee joint.

According to the invention, the multidirectional mobilizer of the knee is incorporating three motion units: knee flexion and extension motion unit; internal and external rotation motion unit of the knee; and a multidirectional patellar mobilization unit (lower-upper and medial-lateral), an external support for multidirectional patellar mobilizer and a control unit.

The flexion and extension motion unit is comprised of a mechanical and electronic structure on which the leg rests during use thereof. The structure for the leg has at least two joints, one in the proximal region proximal of the femur (region of the hip) and another in the distal region of the femur (knee region), enabling the performance of flexion and extension motion of the leg. The structure has at least two supports to support the leg located at the back region of the thigh and at the back region of the leg (calf). The flexion and extension motion are performed passively from a motor coupled to the longitudinal axis of the unit.

The internal and external rotation motion unit of the knee is comprised of a structure with a support for the leg and a support for the foot region (sole region) coupled to the knee flexion and extension motion unit. The leg support may be the same support for the leg of the flexion and extension motion unit with the addition of rotation on the longitudinal axis and of a leg fastening strap by the back part, enabling the performance of the internal and external rotation motion of the knee. The support for the sole region enables the comfortable positioning of the foot to the structure and the fastening of the motor for performing the rotation motion. The body of the motor is fastened to the structure of the flexion and extension motion unit and its axis is coupled to a trapezoidal screw of the internal and external rotation motion unit.

The multidirectional patellar mobilization unit is comprised of a receptacle on which a retainer and an actuating element are disposed. The retainer is comprised of a rigid or semi-rigid structure that is positioned on the upper part of the knee and is coupled to the flexion and extension motion unit through fastening straps with height adjustment. The actuating element is comprised of a set of inflatable cuffs disposed such that the actuating element comes into contact with the kneecap region, but does not exert pressure thereon whilst not in operation. The set of inflatable cuffs of the actuating element is inflated and emptied according to the programming of the system (detailed ahead) from a fluid pump. When the chambers are driven individually, the actuating element comes into contact with the side, lower and back edges of the kneecap and, from the pressure exerted on the edge, the multidirectional motions (frontal-upper and medial-lateral) are performed. The multidirectional patellar mobilization unit can be used in an integrated manner with the flexion and extension motion unit from the straps and side clips or used independently with the assistance of the support for multidirectional patellar mobilizer.

The support for multidirectional patellar mobilizer is comprised of a single element that enables the coupling of the multidirectional patellar mobilizer in a manner similar to the flexion and extension motion unit from the side clip straps, but without the flexion-extension and rotation motion elements. The support for multidirectional patellar mobilizer enables the leg to be fastened at the suitable knee extension position for performing patellar mobilization. The structure has a support for the leg and thigh, on which the leg is fastened with two straps, one for each region, so as to maintain the leg in the extension position during mobilization.

The control unit is comprised of an electro-electronic and pneumatic system. The electro-electronic system is characterized by performing the control of the operation of the device, in an automated manner and from the commands and parameters added by the user. The following are parts of the electro-electronic system: the activation drivers of the motors for performing flexion, extension and rotation movements; the activation drivers of the fluid pump and valves for performing lower-upper and medial-lateral patellar mobilization motion. The power source and the man-machine interface for controlling the adjustment parameters of the device are also parts of the electro-electronic system. The fluid pumps, the valves, the inflatable cuffs and the pneumatic connections are parts of the pneumatic system.

The description below and accompanying drawings, for example, will clarify the understanding of the object of the present application.
Figure 1 presents a schematic drawing of the three motion units integrated in perspective.
Figure 2 presents a schematic drawing of the three motion units integrated in side view.

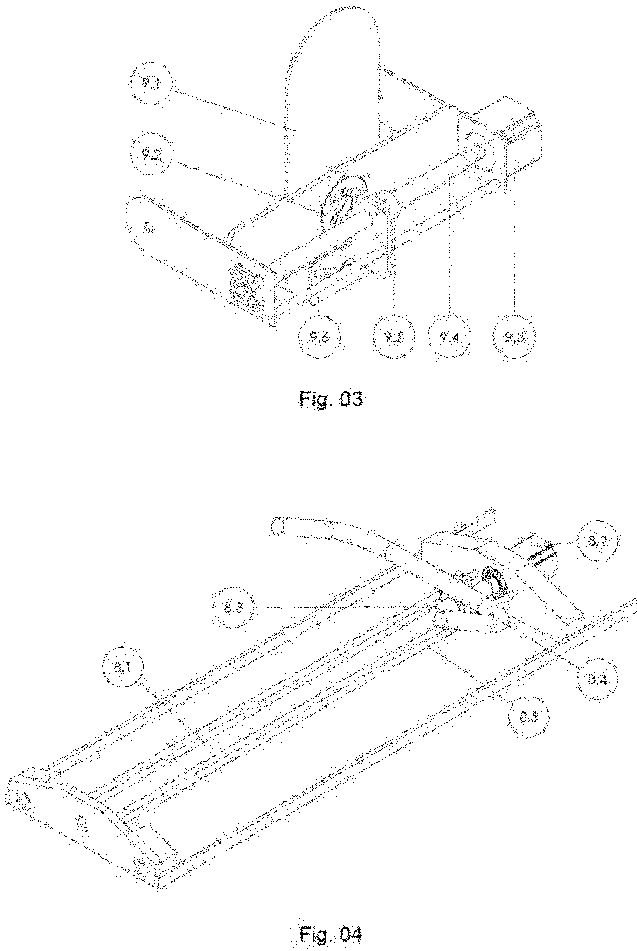

Figure 3 presents a detailed schematic drawing of the rotation device in perspective.
Figure 4 presents a detailed schematic drawing of the flexion and extension device.

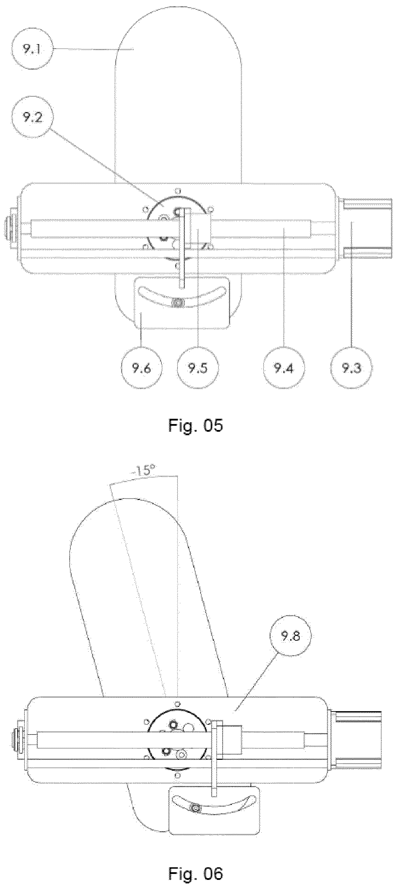

Figure 5 presents a detailed schematic drawing of the rotation device in side view.
Figure 6 presents a detailed schematic drawing of the rotation device with internal rotation.

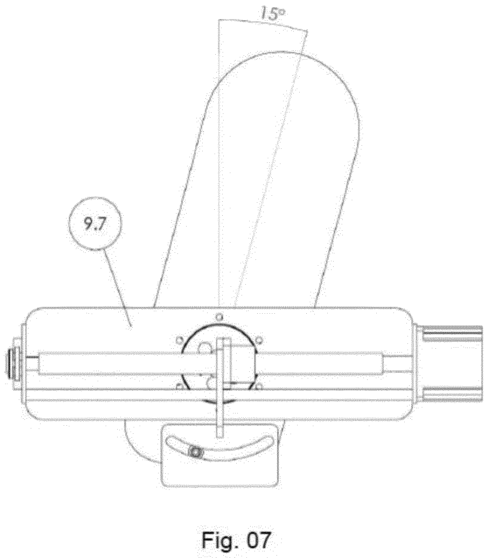

Figure 7 presents a detailed schematic drawing of the rotation device with external rotation.
Figure 8 presents a schematic drawing of the patellar mobilizer and external support for the patellar mobilizer.
Figure 9 presents a detailed schematic drawing of the inflatable cuff container.

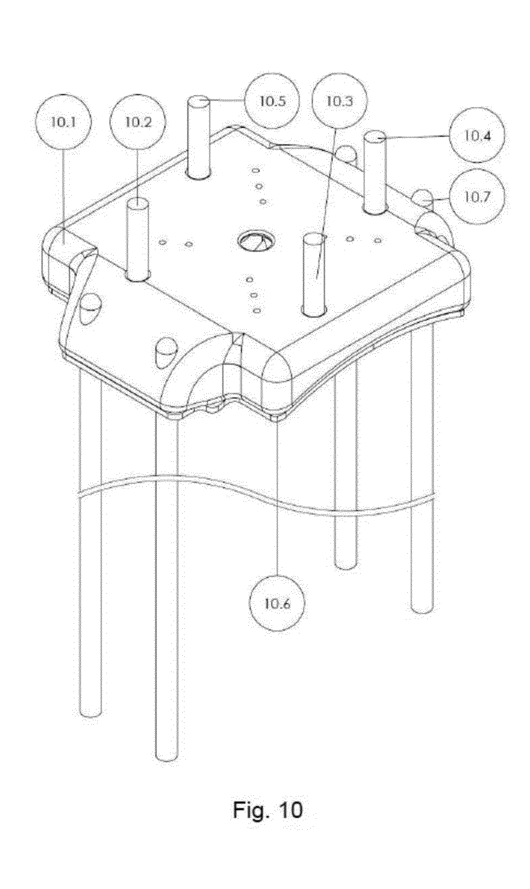

Figure 10 presents a detailed schematic drawing of the inflatable cuff container with side support rods.

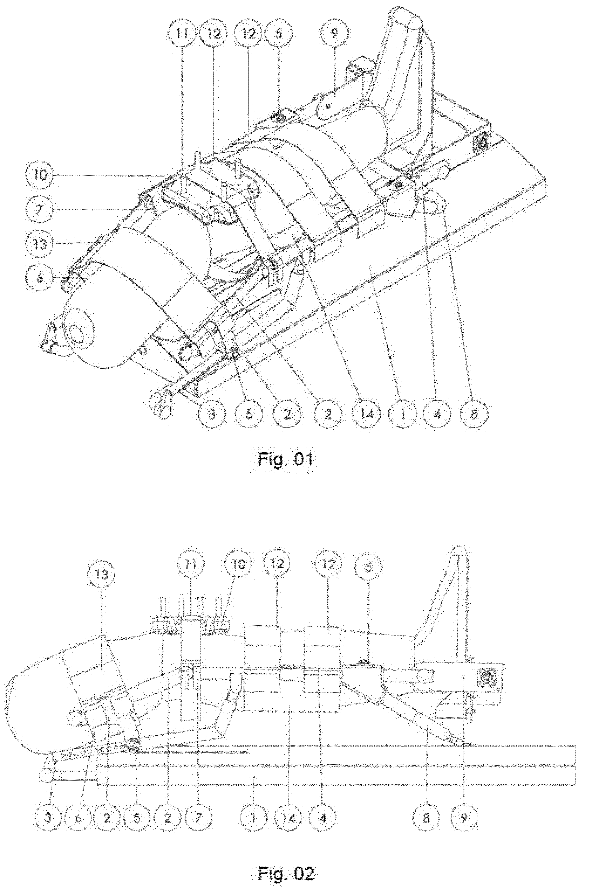

As illustrated in the drawings, the multidirectional patellar mobilization equipment is comprised of three motion units, according to Figure 1, which are integrated into a single apparatus responsible for synchronizing the motions applied in the specific treatment of the patient. The position of use of the apparatus is compulsorily horizontal, with the base (1) positioned parallel to the horizontal plane, enabling the accommodation of the leg of the patient such that the hip remains near the proximal adjustment combination (3) and joint links (2) and that the thigh is supported on the back support of the thigh (6) and the calf is supported on the back support (14) of the leg of the patient. For due accommodation of the leg and, considering the different anatomies and statures of the users, the distal adjustment combination (4) should be adjusted through the device for adjustment locking (5) so that the sole of the foot of said leg of the patient to be supported on a foot support (9.1). On the base (1) there are inserted the electronic and pneumatic control systems needed for the due activation of the electro-mechanical and pneumatic parts of the multidirectional mobilizer of the knee, as well as the man-machine interface through the local display (for example: display touchscreen, alphanumerical, etc.) or remote display (for example: smartphone).

The motion unit (8) for flexion and extension is comprised by the base (1) that is common to all the units, on which there are inserted a motor (8.2) coupled to a trapezoidal screw thread (8.1) and a displacement assembly (8.3), the displacement assembly is directed by guides (8.5) and connected to an articulated U-shaped tube (8.4). When the motor (8.2) is driven the trapezoidal screw thread (8.1) transmits in use a rotation to the displacement assembly (8.3) which moves along the screw, the guides (8.5) restrict the motion of the displacement assembly (8.3) only in a linear direction, upon moving, the assembly connected to the articulated U-shaped tube (8.4) which is part of a mechanism formed by tubes (3, 4) and links (2), transfers the motion of one degree of freedom to two degrees of freedom whereby flexing the knee forward and extending it upon return.

The internal and external rotation motion unit (9) for a knee of said leg of the patient is comprised of the base (1) which provides suitable support for the leg through the back support of the thigh (6), of the back support (14) of the leg of the patient and on a foot support (9.1). The foot support (9.1) has a tightening strap for tibia (12) which is buckled at the distal region of the tibia in order to maintain the tibia in a neutral position relative to the internal-external rotation. The rotation device of the tibia is comprised of the foot support (9.1) coupled to a mobile flange of a turning ring (9.2), which is fastened to one of the plates of the system and a step motor (9.3) coupled to a trapezoidal screw (9.4) that transmits the rotation motion to a displacement assembly (9.5) fastened to the turning guide (9.6). To perform the operation of rotation of the tibia the foot support (9.1) is coupled to the mobile flange of the turning ring (9.2) while the fastened flange of the turning ring (9.2) is fastened on the plate of the system, whereby the foot support turns freely while fastened on the plate. The foot support (9.1) is further connected by a guide pin to a slot on the guide turning plate (9.6) which is fastened to the displacement assembly (9.5), accordingly, when the step motor (9.3) is driven and turns the trapezoidal screw (9.4) displacing the displacement assembly (9.5) the guide pin of the foot support displaces radially inside the slot of the guide plate. In advancing the displacement assembly, the tibia turns internally (counterclockwise) and upon retreating, it turns externally (clockwise) (9.7 and 9.8).

The multidirectional patellar mobilization unit (10) is comprised of an inflatable cuff container (10.1) which is coupled to the base (1) through a second tightening strap (11). In the lower part of the inflatable cuff container (10.1) a set of inflatable cuffs is located, comprised of at least one inflatable cuff. The representation (10.2, 10.3, 10.4, 10.5) shows the combination with four inflatable cuffs distributed in a way so that in use they surround the knee joint. The inflatable cuffs are inserted inside internal containment chambers (10.6) upon containing the cuffs so as to control the direction of the fluid filling in the direction of the desired patellar mobilization (lower-back or medial-lateral). The fluid ducts of the cuffs are positioned on the sides of the inflatable cuff container (10.1), enabling the pneumatic connection with the base (1) through the pneumatic connectors (1).

This constructive form enables the patellar mobilization device to be used individually, that is, a multidirectional patellar mobilization unit can be used in isolation with the assistance of the external support (15) for the multidirectional patellar mobilization unit (10) which is comprised of the base (1) for leg support and fastening the external support (15), side fastening rods (10.7) and fastening straps for the thigh (16) and of the leg (18), wherein the equipment further comprises an electronic and pneumatic control unit (19) which has a man-machine interface through a local or remote display (20). The inflatable cuff container (10.1) is coupled to the external support (15) for the multidirectional patellar mobilization unit (10) by means of the side fastening rods (10.7) that have height adjustment by means of clips, butterflies or the like enabling the use in different anatomies of the knee. The external support (15) for multidirectional patellar mobilizer contains an electronic and pneumatic control unit (19) specific for individualized use of the multidirectional patellar mobilizer. The electronic and pneumatic control unit (19) is connected to the inflatable cuffs (10.2, 10.3, 10.4, 10.5) through the pneumatic connectors (21) through which the flow of fluid enters and leaves the device through the valves and fluid pump.

## Claims

1. EQUIPMENT FOR PASSIVE MOTION OF A KNEE JOINT comprised of three motion units integrated into a single apparatus responsible for synchronizing motions applied in a specific treatment of a patient with dysfunction of the knee, **characterized by**
the three motion units being, respectively:
a motion unit (8) for flexion and extension,
an internal and external rotation motion unit (9) for the knee of a leg of the patient, and
a multidirectional patellar mobilization unit (10),
wherein the three motion units are mounted on a base (1); further comprising an external support (15) for the multidirectional patellar mobilization unit(10) ;
Wherein said base (1) is positioned parallel to a horizontal plane, so as to enable accommodation of said leg of the patient such that the hip of the patient remains near a proximal adjustment assembly (3) and joint links (2), and such that the thigh of said leg of the patient is supported on a back support for the thigh (6) and the calf of said leg of the patient is supported on a back support (14) for said leg of the patient;
wherein the equipment further comprises an assembly (4) for distal adjustment adjustable by means of a device for adjustment locking (5) so as to enable the sole of the foot of said leg of the patient to be supported on a foot support (9.1);
Wherein said base (1) discloses electronic, pneumatic control and man-machine interface systems through local or remote display;
wherein said motion unit (8) for flexion and extension comprises a motor (8.2) coupled to a trapezoidal screw thread (8.1) and a displacement assembly (8.3),
the displacement assembly (8.3)-being directed in use by guides (8.5) and connected to an articulated U-shaped tube (8.4), such that when the motor (8.2) is driven the trapezoidal screw thread (8.1) transmits in use a rotation to the displacement assembly (8.3),
such that the displacement assembly (8.3) moves in use along the trapezoidal screw thread (8.1), and
such that the guides (8.5) enable the motion of the displacement assembly (8.3) only in a linear direction;
wherein the connection of the displacement assembly (8.3) to the articulated U- shaped tube (8.4) makes up a mechanism formed by tubes (3, 4) and links (2), such that upon moving it transfers the motion of one degree of freedom to two degrees of freedom;
wherein said internal and external rotation motion unit (9) for a knee of said leg of the patient comprises on a foot support (9.1) a first tightening strap (12) for the tibia of said leg of the patient, disposed so that it can be buckled to a distal region of the tibia, and
wherein said foot support (9.1) provides a mobile flange of a turning ring (9.2) which is fastened to one of more than one plates of the system, and
wherein said internal and external rotation motion unit (9) further comprises a step motor (9.3) coupled to a trapezoidal screw (9.4) which transmits a rotation motion to a displacement assembly (9.5) fastened to a turning guide (9.6), and
wherein said foot support (9.1) is coupled to the mobile flange of the turning ring (9.2) and the fastened flange of the turning ring (9.2) is fastened on the plates of the system, such that in use the foot support (9.1) turns freely while fastened on the plates of the system, and
wherein said foot support (9.1) is connected to the displacement assembly (9.5) in such a manner that when the step motor (9.3) is driven in use it makes the trapezoidal screw (9.4) turn, thereby displacing the displacement assembly (9.5), and a guide pin of the foot support (9.1) displaces radially inside of a slot of a guide plate;
wherein said multidirectional patellar mobilization unit (10) is comprised by inflatable cuff container (10.1) which is coupled to the base (1) by means of a second tightening strap (11), and
wherein on a lower part of the inflatable cuff container (10.1) there is disposed a set of inflatable cuffs (10.2, 10.3, 10.4, 10.5) distributed so that in use they surround the knee joint of said leg of the patient , and
wherein the inflatable cuffs are inserted inside internal containment chambers (10.6) into the inflatable cuff container (10.1) in such a manner that they control a direction of a fluid filling, and
wherein fluid ducts of the cuffs are positioned on the sides of the inflatable cuff container (10.1 ), creating a pneumatic connection with the base (1) by means of pneumatic connectors (21);
wherein said external support (15) for the multidirectional patellar mobilization unit (10) is comprised by:
the base (1) for leg support and for fastening the external support (15),
side fastening rods (10.7), and
fastening straps for the thigh (16) and for the leg (18);
wherein the equipment further comprises an electronic and pneumatic control unit (19) which provides a man-machine interface through a local or remote display (20 ;
wherein the inflatable cuff container (10.1) is coupled to the external support (15) for the multidirectional patellar mobilization unit (10) by means of the side fastening rods (10.7) which allows for height adjustment by means of clips;
wherein said external support (15) for multidirectional patellar mobilization unit (10) allows for the electronic and pneumatic control unit (19) to be connected to the set of inflatable cuffs (10.2, 10.3, 10.4, 10.5) bymeans of pneumatic connectors (21).

## Patentansprüche

1. Vorrichtung zur passiven Bewegung eines Kniegelenks, die aus drei Bewegungseinheiten besteht, welche in ein einzelnes Gerät integriert sind, das zum Synchronisieren von Bewegungen verantwortlich ist, die bei einer spezifischen Behandlung eines Patienten mit Kniefunktionsstörungen angewendet werden, **dadurch gekennzeichnet, dass**
die drei Bewegungseinheiten jeweils Folgendes sind:
eine Bewegungseinheit (8) zur Beugung und Streckung,
eine interne und externe Rotationsbewegungseinheit (9) für das Knie eines Beins des Patienten und
eine multidirektionale Patellamobilisierungseinheit (10),
wobei die drei Bewegungseinheiten auf einer Basis (1) montiert sind; ferner umfassend eine externe Stütze (15) für die multidirektionale Patellamobilisierungseinheit (10);
wobei die Basis (1) parallel zu einer horizontalen Ebene positioniert ist, um die Aufnahme des Beins des Patienten zu ermöglichen, sodass die Hüfte des Patienten in der Nähe einer proximalen Einstellungsbaugruppe (3) und Gelenken (2) bleibt und sodass die Höhe des Beins des Patienten auf einer Rückenstütze für den Oberschenkel (6) gestützt wird und die Wade des Beins des Patienten auf einer Rückenstütze (14) für das Bein des Patienten gestützt wird;
wobei die Vorrichtung ferner eine Baugruppe (4) zur distalen Einstellung umfasst, die mittels einer Einrichtung zur Einstellungsverriegelung (5) einstellbar ist, um zu ermöglichen, dass die Fußsohle des Beins des Patienten auf einer Fußstütze (9.1) gestützt wird;
wobei die Basis (1) elektronische, pneumatische Steuerungs- und Mensch-Maschine-Schnittstellensysteme über Lokal- oder Fernanzeige offenbart;
wobei die Bewegungseinheit (8) zur Beugung und Streckung einen Motor (8.2) umfasst, der an ein Trapezspindelgewinde (8.1) und eine Verschiebungsbaugruppe (8.3) gekoppelt ist,
wobei die Verschiebungsbaugruppe (8.3) bei der Verwendung durch Führungen (8.5) geleitet wird und mit einem gelenkigen U-förmigen Schlauch (8.4) verbunden ist, sodass, wenn der Motor (8.2) angetrieben wird, das Trapezspindelgewinde (8.1) bei der Verwendung eine Rotation auf die Verschiebungsbaugruppe (8.3) überträgt,
sodass sich die Verschiebungsbaugruppe (8.3) bei der Verwendung entlang des Trapezspindelgewindes (8.1) bewegt und
sodass die Führungen (8.5) die Bewegung der Verschiebungsbaugruppe (8.3) nur in einer linearen Richtung ermöglichen;
wobei die Verbindung der Verschiebungsbaugruppe (8.3) mit dem gelenkigen U-förmigen Schlauch (8.4) einen Mechanismus ausmacht, der durch Schläuche (3, 4) und Gelenke (2) gebildet wird, sodass er beim Bewegen die Bewegung eines Freiheitsgrades auf zwei Freiheitsgrade überträgt;
wobei die interne und externe Rotationsbewegungseinheit (9) für ein Knie des Beins des Patienten auf einer Fußstütze (9.1) einen ersten Spanngurt (12) für das Schienbein des Beins des Patienten umfasst, der so angeordnet ist, dass er an eine distale Region des Schienbeins geschnallt werden kann, und
wobei die Fußstütze (9.1) einen mobilen Flansch eines Drehrings (9.2) bereitstellt, der an eine oder mehr als eine Platte des Systems befestigt ist, und
wobei die interne und externe Rotationseinheit (9) ferner einen Schrittmotor (9.3) umfasst, der an eine Trapezspindel (9.4) gekoppelt ist, die eine Rotationsbewegung auf eine Verschiebungsbaugruppe (9.5) überträgt, welche an einer Drehführung (9.6) befestigt ist, und
wobei die Fußstütze (9.1) an den mobilen Flansch des Drehrings (9.2) gekoppelt ist und der befestigte Flansch des Drehrings (9.2) an den Platten des Systems befestigt ist, sodass sich die Fußstütze (9.1) bei der Verwendung frei dreht, während sie an den Platten des Systems befestigt ist, und
wobei die Fußstütze (9.1) mit der Verschiebungsbaugruppe (9.5) in einer solchen Weise verbunden ist, dass, wenn der Schrittmotor (9.3) bei der Verwendung angetrieben wird, die Trapezspindel (9.4) gedreht wird, wodurch die Verschiebungsbaugruppe (9.5) verschoben wird, und sich ein Führungsstift der Fußstütze (9.1) radial innerhalb eines Schlitzes einer Führungsplatte verschiebt;
wobei die multidirektionale Patellamobilisierungseinheit (10) aus einem aufblasbaren Manschettenbehälter (10.1) besteht, der an die Basis (1) mittels eines zweiten Spanngurtes (11) gekoppelt ist, und
wobei auf einem unteren Teil des aufblasbaren Manschettenbehälters (10.1) ein Satz aufblasbarer Manschetten (10.2, 10.3, 10.4, 10.5) angeordnet ist, die so verteilt sind, dass sie bei der Verwendung das Kniegelenk des Beins des Patienten umgeben, und
wobei die aufblasbaren Manschetten in interne Eindämmungskammern (10.6) in den aufblasbaren Manschettenbehälter (10.1) in einer solchen Weise eingeführt sind, dass sie eine Richtung einer Fluidfüllung steuern, und
wobei Fluidkanäle der Manschetten an den Seiten des aufblasbaren Manschettenbehälters (10.1) positioniert sind, wobei eine pneumatische Verbindung mit der Basis (1) mittels pneumatischer Verbinder (21) erzeugt wird;
wobei die externe Stütze (15) für die multidirektionale Patellamobilisierungseinheit (10) aus Folgendem besteht:
der Basis (1) zur Beinstütze und zum Befestigen der externen Stütze (15),
seitlichen Befestigungsstäben (10.7) und
Befestigungsgurten für den Oberschenkel (16) und für das Bein (18);
wobei die Vorrichtung ferner eine elektronische und pneumatische Steuereinheit (19) umfasst, die eine Mensch-Maschine-Schnittstelle über eine Lokal- oder Fernanzeige (20) bereitstellt;
wobei der aufblasbare Manschettenbehälter (10.1) an die externe Stütze (15) für die multidirektionale Patellamobilisierungseinheit (10) mittels der Befestigungsstäbe (10.7) gekoppelt ist, was eine Höheneinstellung mittels Klammern ermöglicht;
wobei die externe Stütze (15) für die multidirektionale Patellamobilisierungseinheit (10) ermöglicht, dass die elektronische und pneumatische Steuereinheit (19) mit dem Satz aufblasbarer Manschetten (10.2, 10.3, 10.4, 10.5) mittels pneumatischer Verbinder (21) verbunden wird.

## Revendications

1. Équipement pour le mouvement passif d'une articulation du genou composé de trois unités de mouvement intégrées dans un seul appareil responsable de la synchronisation des mouvements appliqués dans un traitement spécifique d'un patient présentant un dysfonctionnement du genou, **caractérisé en ce que**
les trois unités de mouvement sont, respectivement :
une unité de mouvement (8) pour la flexion et l'extension,
une unité de mouvement de rotation interne et externe (9) pour le genou d'une jambe du patient, et
une unité de mobilisation rotulienne multidirectionnelle (10),
dans lequel les trois unités de mouvement sont montées sur une base (1) ; comprenant en outre un support externe (15) pour l'unité de mobilisation rotulienne multidirectionnelle (10) ;
dans lequel ladite base (1) est positionnée parallèlement à un plan horizontal, de manière à permettre l'accommodation de ladite jambe du patient de telle sorte que la hanche du patient reste proche d'un ensemble de réglage proximal (3) et de liaisons articulaires (2), et de telle sorte que la cuisse de ladite jambe du patient soit soutenue sur un support dorsal pour la cuisse (6) et le mollet de ladite jambe du patient soit soutenu sur un support dorsal (14) pour ladite jambe du patient :
dans lequel l'équipement comprend en outre un ensemble (4) de réglage distal réglable au moyen d'un dispositif de verrouillage de réglage (5) de manière à permettre à la plante du pied de ladite jambe du patient d'être appuyée sur un support de pied (9.1) ;
dans lequel ladite base (1) divulgue des systèmes de contrôle électronique, pneumatique et d'interface homme-machine par affichage local ou distant ;
dans lequel ladite unité de mouvement (8) pour la flexion et l'extension comprend un moteur (8.2) couplé à un filetage trapézoïdal (8.1) et un ensemble de déplacement (8.3),
l'ensemble de déplacement (8.3) étant dirigé en utilisation par des guides (8.5) et relié à un tube articulé en forme de U (8.4), de telle sorte que lorsque le moteur (8.2) est entraîné, le filetage trapézoïdal (8.1) transmet en utilisation une rotation à l'ensemble de déplacement (8.3),
de telle sorte que l'ensemble de déplacement (8.3) se déplace en utilisation le long du filetage trapézoïdal (8.1), et
de telle sorte que les guides (8.5) permettent le mouvement de l'ensemble de déplacement (8.3) uniquement dans une direction linéaire ;
dans lequel la connexion de l'ensemble de déplacement (8.3) au tube articulé en forme de U (8.4) constitue un mécanisme formé de tubes (3, 4) et de liaisons (2), de telle sorte que lors du déplacement, il transfère le mouvement d'un degré de liberté à deux degrés de liberté ;
dans lequel ladite unité de mouvement de rotation interne et externe (9) pour un genou de ladite jambe du patient comprend, sur un support de pied (9.1), une première sangle de serrage (12) pour le tibia de ladite jambe du patient, disposée de manière à pouvoir être attachée à une région distale du tibia, et
dans lequel ledit support de pied (9.1) fournit une bride mobile d'une bague tournante (9.2) qui est fixée à une ou plusieurs plaques du système, et
dans lequel ladite unité de mouvement de rotation interne et externe (9) comprend un moteur pas à pas (9.3) couplé à une vis trapézoïdale (9.4) qui transmet un mouvement de rotation à un ensemble de déplacement (9.5) fixé à un guide de rotation (9.6), et
dans lequel ledit support de pied (9.1) est couplé à la bride mobile de la bague tournante (9.2) et la bride fixée de la bague tournante (9.2) est fixée sur les plaques du système, de telle sorte qu'en utilisation le support de pied (9.1) tourne tout en étant fixé sur les plaques du système, et
dans lequel ledit support de pied (9.1) est relié à l'ensemble de déplacement (9.5) de telle manière que lorsque le moteur pas à pas (9.3) est entraîné en utilisation, il fait tourner la vis trapézoïdale (9.4), déplaçant ainsi l'ensemble de déplacement (9.5), et une broche de guidage du support de pied (9.1) se déplace radialement à l'intérieur d'une fente d'une plaque de guidage ;
dans lequel ladite unité de mobilisation rotulienne multidirectionnelle (10) est composée d'un récipient de manchette gonflable (10.1) qui est couplé à la base (1) au moyen d'une seconde sangle de serrage (11), et
dans lequel sur une partie inférieure du récipient de manchette gonflable (10.1) est disposé un ensemble de manchettes gonflables (10.2, 10.3, 10.4, 10.5) réparties de manière à ce qu'en utilisation elles entourent l'articulation du genou de ladite jambe du patient, et
dans lequel les manchettes gonflables sont insérées à l'intérieur de chambres de confinement internes (10.6) dans le récipient de manchette gonflable (10.1) de telle manière qu'elles contrôlent la direction d'un remplissage de fluide, et
dans lequel des conduits de fluide des manchettes sont positionnés sur les côtés du récipient de manchette gonflable (10.1), créant une connexion pneumatique avec la base (1) au moyen de connecteurs pneumatiques (21) ;
dans lequel ledit support externe (15) pour l'unité de mobilisation rotulienne multidirectionnelle (10) est composé par :
la base (1) pour le support de jambe et pour la fixation du support externe (15),
des tiges de fixation latérales (10.7) et
des sangles de fixation pour la cuisse (16) et pour la jambe (18) ;
dans lequel l'équipement comprend en outre une unité de commande électronique et pneumatique (19) qui fournit une interface homme-machine via un affichage local ou distant (20) ;
dans lequel le récipient de manchette gonflable (10.1) est couplé au support externe (15) pour l'unité de mobilisation rotulienne multidirectionnelle (10) au moyen des tiges de fixation latérales (10.7) qui permettent un réglage en hauteur au moyen d'attaches ;
dans lequel ledit support externe (15) pour unité de mobilisation rotulienne multidirectionnelle (10) permet de connecter l'unité de commande électronique et pneumatique (19) à l'ensemble de manchettes gonflables (10.2, 10.3, 10.4, 10.5) au moyen de connecteurs pneumatiques (21).
